# NEUE EUROPÄISCHE PATENTSCHRIFT

(11) **EP 2 244 996 B2**
(45) Veröffentlichungstag und Bekanntmachung des Hinweises auf die Entscheidung über den Einspruch: **11.11.2015**
(45) Hinweis auf die Patenterteilung: 31.10.2012
(21) Anmeldenummer: 09709624.2
(22) Anmeldetag: 29.01.2009
(51) Int. Cl.: C07C 29/50, C07C 51/367, C07C 51/487, C07C 67/08, C07C 59/01, C07C 69/675, C07C 45/33

(54) **VERFAHREN ZUR HERSTELLUNG VON 6-HYDROXYCAPRONSÄUREESTERN**
METHOD FOR PRODUCING 6-HYDROXY HEXANOIC ACID ESTERS
PROCEDE DE PRODUCTION D'ESTERS D'ACIDE 6-HYDROXYCAPRONIQUE

(30) Priorität: 15.02.2008 EP 08101692
(43) Veröffentlichungstag der Anmeldung: 03.11.2010
(73) Patentinhaber: BASF SE, 67056 Ludwigshafen (DE)
(72) Erfinder: PINKOS, Rolf, 67098 Bad Dürkheim (DE); SIRCH, Tilman, 67105 Schifferstadt (DE); Dr. TEBBEN, Gerd-Dieter, 68239 Mannheim (DE); FISCHER, Rolf-Hartmuth, 69121 Heidelberg (DE)
(86) Internationale Anmeldenummer: PCT/EP2009/051000
(87) Internationale Veröffentlichungsnummer: WO 2009/100989

(56) Entgegenhaltungen:
- EP-A1- 0 847 979
- DE-A1- 1 951 250
- DE-A1- 2 358 460
- DE-A1- 3 823 695
- US-A- 3 933 930
- US-A- 5 981 769
- US-A- 6 063 958

## Beschreibung

Die vorliegende Anmeldung betrifft ein verbessertes Verfahren zur Herstellung von 6-Hydroxycapronsäureestern aus den Nebenprodukt-Gemischen, die bei der Oxidation von Cyclohexan zu Cyclohexanol und Cyclohexanon mit Sauerstoff oder Sauerstoff enthaltenden Gasgemischen anfallen.

6-Hydroxycapronsäure und die Ester der 6-Hydroxycapronsäure lassen sich zu ε-Caprolacton cyclisieren. ε-Caprolacton bzw. die daraus durch Polyaddition gebildeten Polycaprolactone dienen zur Herstellung von Polyurethanen.

Es ist bekannt, die Oxidation von Cyclohexan zu Cyclohexanol und Cyclohexanon entweder einstufig in Gegenwart von Cobaltverbindungen als Katalysatoren oder zweistufig durchzuführen.

Bei der zweistufigen Fahrweise wird im ersten Schritt ohne Katalysator gearbeitet. Das dabei gebildete Cyclohexylhydroperoxid wird im zweiten Schritt in Gegenwart eines Cobalt-Katalysators zu Cyclohexanol und Cyclohexanon umgesetzt wie es von Arpentinier et al. in The technology of catalytic oxidations, Editions Technip 2001, Seite 227, 1. und 3. Absatz beschrieben ist.

Aus DE-A 2 358 460 ist auch schon bekannt, in wässrigen oder organischen Lösungsmitteln gelöstes Cyclohexylhydroperoxid in Gegenwart von Edelmetall-Katalysatoren zu Cyclohexanol zu hydrieren.

DE-A 1 951 250 und EP-B 847 979 lehren, wie man aus den Produkten derzweistufigen Cyclohexanol-Herstellung 6-Hydroxycapronsäure gewinnen kann. Hierzu wird Cyclohexan in der Flüssigphase in Abwesenheit eines Katalysators oxidiert. Der Oxidationsaustrag, der hauptsächlich Cyclohexylhydroperoxid, Cyclohexanol, Cyclohexanon und Oxidationsfolgeprodukte wie 6-Hydroperoxycapronsäure, 6-Hydroxycapronsäure, Adipinsäure, Bernsteinsäure, Glutarsäure und auch 5-Formylvaleriansäure wie in Beispiel 1, Zeile 19 und Beispiel 3, Zeile 47 und in Abschnitt [0005] von EP-B 847 979 unter den Begriffen "semialdehyde adipique" oder "acide formyl-5-valerique" beschrieben, enthält, wird mit Wasser versetzt. Das dann zweiphasige Reaktionsgemisch wird in eine organische und eine wässrige Phase getrennt.

Die organische Phase wird, wie schon beschrieben, durch Deperoxidation mit Metallverbindungen oder durch katalytische Hydrierung in Cyclohexanol und Cyclohexanon umgewandelt.

Die Wasserphase enthält neben 6-Hydroperoxycapronsäure 6-Hydroxycapronsäure, 5-Formylvaleriansäure, Adipinsäure, Bernsteinsäure und Glutarsäure.

Um 6-Hydroperoxycapronsäure in 6-Hydroxycapronsäure umzuwandeln, wird die Wasserphase nach DE-A 1 951 250 in Gegenwart von Palladium-, Rhodium- oder Platinkatalysatoren bei 15 bis 130 °C, vorzugsweise 50 bis 100 °C und Drucken von 2 bis 20 bar hydriert.

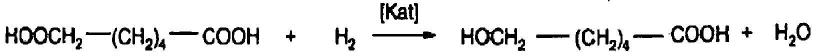

Die genannten Metalle können auf Träger wie SiO2, Al2O3, Aktivkohle oder Alumosilikate aufgebracht sein. In den drei Ausführungsbeispielen wird mit einem Trägerkatalysator gearbeitet, der 10 Gew.-% Palladium auf Aktivkohle enthält.

Nachteilig bei der Hydrierung von 6-Hydroperoxycapronsäure gemäß DE-A 1 951 250 ist, dass die in der Wasserphase enthaltene 5-Formylvaleriansäure bei 15 bis 130 °C und 2 bis 20 bar in Gegenwart der genannten Edelmetall-Katalysatoren nicht vollständig zu 6-Hydroxycapronsäure hydriert wird. Bei der Veresterung der 6-Hydroxycapronsäure und anschließender destillativer Reinigung erhält man ein Gemisch aus 6-Hydroxycapronsäureestern, 5-Formylvaleriansäureestern und deren Acetale, da alle Ester sehr ähnliche Dampfdrücke besitzen. Eine vollständige destillative Abtrennung der Formylvaleriansäureester wäre nur mit hohem Destillationsaufwand, verbunden mit hohen Energiekosten, möglich.

Es bestand daher die Aufgabe, Hydrierkatalysatoren und Hydrierbedingungen bereitzustellen, mit denen 6-Hydroperoxycapronsäure und 5-Formylvaleriansäure mit hohen Ausbeuten zu 6-Hydroxycapronsäure hydriert werden können.

Diese Aufgabe wird gelöst durch ein Verfahren zur Herstellung von 6-Hydroxycapronsäureestern wobei man
a) Cyclohexan mit molekularem Sauerstoff oder Gemischen aus molekularem Sauerstoff und unter den Reaktionsbedingungen inerten Gasen zu einem Reaktionsgemisch oxidiert, das als Hauptkomponenten Cyclohexylhydroperoxid, Cyclohexanol, Cyclohexanon, unumgesetztes Cyclohexan, 6-Hydroperoxycapronsäure, 6-Hydroxycapronsäure, 5-Formylvaleriansäure und α,ω- Dicarbonsäuren mit vier bis sechs Kohlenstoffatomen enthält,
b) das Reaktionsgemisch aus Schritt a) nach Zugabe von Wasser in eine organische, Cyclohexan und die Cyclohexan-Verbindungen enthaltende und eine wässrige, die Carbonsäuren enthaltende Phase trennt,
c) die wässrige Phase aus b) katalytisch hydriert,
d) die in der wässrigen Phase enthaltenen Carbonsäuren mit einem 1 bis 10 C-Atome enthaltendem Alkohol zu den entsprechenden Carbonsäureestern umsetzt und
e) aus dem Veresterungsgemisch aus Schritt d) durch Destillation 6-Hydroxycapronsäureester gewinnt,
wobei bei der katalytischen Hydrierung in Schritt c) mindestens 5-Formylvaleriansäure zu 6-Hydroxycapronsäure hydriert wird und als Katalysatormetall für die Hydrierung in Schritt c) mindestens ein Metall, ausgewählt aus der Gruppe von Ruthenium und Rhenium verwendet wird.

Vorteilhaft ist das erfindungsgemäße Verfahren wenn der Schritt a) in Anwesenheit eines Katalysators durchgeführt wird.

Vorteilhaft ist das erfindungsgemäße Verfahren, wenn der Schritt a) in Abwesenheit eines Katalysators durchgeführt wird.

Vorteilhaft ist das erfindungsgemäße Verfahren, wenn man in Schritt c) neben 5-Formylvaleriansäure auch 6-Hydroperoxycapronsäure zu 6-Hydroxycapronsäure hydriert.

Vorteilhaft ist das erfindungsgemäße Verfahren, wenn man die wässrige, die Carbonsäuren enthaltende Phase aus Schritt b) zur Entfernung von Restmengen Cyclohexylhydroperoxid, Cyclohexanol und Cyclohexanon mit einem aliphatischen, cycloaliphatischen oder aromatischen Kohlenwasserstoff extrahiert.

Vorteilhaft ist das erfindungsgemäße Verfahren, wenn man 6-Hydroperoxycapronsäure und 6-Hydroxycapronsäure aus der wässrigen Phase von Schritt b) mit einem organischen, inerten Lösungsmittel aus der Wasserphase extrahiert.

Vorteilhaft ist das erfindungsgemäße Verfahren, wenn die wässrige, die Carbonsäuren enthaltende Phase aus Schritt b) durch Abdestillieren von Wasser konzentriert wird und die in fester Form ausfallenden Carbonsäuren abgetrennt werden.

Vorteilhaft ist das erfindungsgemäße Verfahren, wenn man die Hydrierung in Schritt c) bei Temperaturen über 100 bis 200°C und Drucken von 1 bis 100 durchführt.

Bei dem erfindungsgemäßen Verfahren kann aus der organischen Phase des Schrittes b) auch noch Cyclohexanol und Cylohexanon gewonnen werden. Die zweistufige Herstellung von Cyclohexanol und Cyclohexanon aus Cyclohexan ist bekannt. Aus Arpentinier et al., The technology of catalytic oxidation, Edition Technip 2001, Seite 227, dritter Absatz geht hervor, dass man im ersten Schritt Hydroperoxide herstellt. Dabei arbeitet man in Abwesenheit von Katalysatoren. Im zweiten Schritt wird das abgetrennte Cyclohexylhydroperoxid in Gegenwart eines Co-Katalysators zu Cyclohexanol und Cyclohexanon zersetzt. Das Verfahren arbeitet mit einem Cyclohexan-Umsatz von 4 bis 5 %, einer CyclohexanollCyclohexanon-'Selektivität im Bereich von 82 bis 86 % und einem Cyclohexanol/Cyclohexanon-Verhältnis von 0,4.

Aus DE-A 1 951 250, Seite 2, zweiter Absatz ist bekannt, die Katalysator-freie Oxidation von Cyclohexan mit kurzen Verweilzeiten und verhältnismäßig niedrigen Temperaturen in Apparaturen durchzuführen, deren Oberfläche die Zersetzung von Hydroperoxiden nicht katalysiert.

FR-A 1 505 363 enthält auf Seite 4, linke Seite, zweiter Absatz eine Vorschrift zur Katalysator-freien Oxidation von Cyclohexan. Dabei wird Cyclohexan mit Luft oxidiert, deren Sauerstoff-Gehalt auf 13 bis 14 Volumenprozent abgereichert wurde. Gearbeitet wird bei 170 bis 180 °C und einem Druck von 18 bar in einer Apparatur aus nichtoxidierbarem Stahl, die mit Pyrophosphat passiviert wurde. Die Oxidation wird beendet, sobald das Oxidat 4 Gew.-% an Produkten enthält, die höher als Cyclohexan sieden.

Es ist auch möglich, Cyclohexan-Oxidate zu verwenden, die in Gegenwart von 0,1 bis 300 ppm, bevorzugt 0,1 bis 200 ppm, besonders bevorzugt 0,1 bis 100 ppm eines Deperoxidationskatalysators, bezogen auf Cyclohexan, erhalten wurden. Als Deperoxidationskatalysatoren sind Cobaltverbindungen geeignet, die in Cyclohexan unter den Oxidationsbedingungen löslich sind. Beispiele hierfür sind Cobalt-Salze von Carbonsäuren, wie z. B. Cobaltnaphthenat, Cobaltstearat oder Cobaltocteat. Mit steigendem Gehalt an Oxidationskatalysator nimmt der Gehalt an Hydroperoxiden im OxidationsAustrag ab.

Die Katalysator-freie Cyclohexan-Oxidation ist gegenüber der katalysierten Cyclohexan-Oxidation bevorzugt.

In dem erfindungsgemäßen Verfahren wird in Schritt b) der Oxidationsaustrag der Cyclohexan-Oxidation, wie in DE-A 1 951 250 beschrieben, mit Wasser versetzt. Nach dem erfindungsgemäßen Verfahren werden hierfür Austräge aus der nicht katalysierten Cyclohexan-Oxidation verwendet. Austräge aus der katalysierten Cyclohexan-Oxidation sind jedoch auch möglich.

Die Oxidationsausträge können durch Abdestillieren von Cyclohexan konzentriert werden. Die Oxidationsausträge enthalten, gegebenenfalls nach Abdestillieren eines Teils des Cyclohexans, mindestens 10 Gew.%, bevorzugt mindestens 30 Gew.-%, besonders bevorzugt mindestens 50 Gew.-% Hydroperoxide, bezogen auf die oxidierten Produkte, die schwerer als Cyclohexan sind.

Der Oxidationsaustrag der Cyclohexan-Oxidation wird gemäß DE-A 1 951 250, in flüssiger Phase, gegebenenfalls nach Abdestillieren von Cyclohexan, bei Temperaturen zwischen 5 und 100 °C, bevorzugt zwischen 15 und 30 °C, unter Eigendruck oder unter Druck mittels eines Inertgases, wenn die gewählte Temperatur über dem Siedepunkt des azeotropen Gemisches Wasser/Cyclohexan liegt, mit der 0,01 bis 10 fachen Gewichts-Menge Wasser, bezogen auf die Menge an Oxidationsaustrag, versetzt.

Der Oxidationsaustrag zerfällt nach Zugabe von Wasser in zwei flüssige Phasen. Eine organische Phase, die Cyclohexylhydroperoxid,-Cyclohexanol, Cyclohexanon und nicht umgesetztes Cyclohexan enthält. Weiterhin eine wässrige Phase, die 6-Hydroperoxycapronsäure, 5-Hydroxyvaleriansäure 6-Hydroxycapronsäure, Adipinsäure, Bernsteinsäure, Glutarsäure und 5-Formylvaleriansäure, Monocarbonsäuren mit 1 bis 6 Kohlenstoffatomen, 1,2- und 1,4-Cyclohexandione und eine Vielzahl von geringen Mengen weiterer Nebenprodukte enthält.

Das zweiphasige flüssige Reaktionsgemisch wird in Schritt b) des erfindungsgemäßen Verfahrens in eine flüssige organische Phase und eine flüssige wässrige Phase getrennt.

Die Umsetzung des Oxidationsaustrags mit Wasser und die anschließende Phasentrennung können diskontinuierlich oder kontinuierlich, bevorzugt kontinuierlich durchgeführt werden.

Die in Schritt b) abgetrennte flüssige organische Phase kann zur Herstellung von Cyclohexanol und Cyclohexanon aufgearbeitet werden. Dafür wird die flüssige organische Phase aus Schritt b) durch Deperoxidation mit Metallverbindungen oder durch katalytische Hydrierung in Gemische aus Cyclohexanol und Cyclohexanon umgewandelt.

Bei der Deperoxidation von Cyclohexylhydroperoxid zu Cyclohexanol und Cyclohexanon wird die flüssige organische Phase mit 1 bis 300 ppm einer Metallverbindung versetzt. Bevorzugt hierbei sind Cobaltsalze von Carbonsäuren, wie z. B. Cobaltnaphthenat oder Cobaltocteat. Gearbeitet wird bei 120 bis 200 °C und Eigendruck des Systems oder Drucken bis zu 5 bar, die durch Inertgase erzeugt werden.

Die Deperoxidation kann diskontinuierlich, bevorzugt aber kontinuierlich durchgeführt werden.

Die Hydrierung von Cyclohexylhydroperoxid zu Cydohexanol/Cyclohexanon-Gemischen kann nach DE-A 2 358 460 in Gegenwart von fein verteilten, suspendierten Katalysatoren auf der Basis der Edelmetalle der 8. Nebengruppe des Periodensystems der Elemente Ruthenium, Rhodium, Palladium, Osmium, Iridium, Platin erfolgen. Bevorzugt sind die Elemente Palladium, Rhodium und Platin. Die Hydrierung wird bei Temperaturen von 20 bis 180 °C, insbesondere 50 bis 100 °C durchgeführt. Der Wasserstoff-Partialdruck beträgt dabei 0,1 bis 50 at, bevorzugt 10 at, insbesondere 1 bis 2 at. In den 12 Ausführungsbeispielen wurden als Katalysatoren 10 % Pd auf Aktivkohle, 4,1 % Pd + 1,1 % Pt auf Aktivkohle, 5 % Pd auf Al₂O₃ und 1 % Pd auf SiO₂ in suspendierter Form eingesetzt. Hydriert wurde bei 30 bis 60 °C und 1 at Wasserstoffpartialdruck in Cyclohexan als Lösungsmittel.

Nach FR-A 1 505 363 auf Seite 2, rechte Spalte, letzter Absatz werden für die katalytische Hydrierung von Cyclohexylhydroperoxid in Cyclohexan als Lösungsmittel Katalysatoren auf der Basis von Palladium, Rhodium, Rhenium und Nickel, bevorzugt von Palladium und Rhodium, verwendet. Die Metalle werden auf saure oder neutrale Träger aufgebracht. Beispiele für derartige Träger sind z. B. Aktivkohle, SiO₂ oder Al₂O₃. Hydriert wird bei 80 bis 100 °C und 10 bis 20 bar. Vor der Hydrierung wird dem Reaktionsgemisch Wasser hinzugefügt. Oberhalb von 100 °C nehmen unerwünschte Nebenreaktionen zu.

Die Hydrierung kann in einem Reaktor oder in mehreren hintereinander geschalteten Reaktoren durchgeführt werden. In einer in FR-A 1 505 363 bevorzugten Ausführungsform wird in einer Kolonne gearbeitet, die einen fest angeordneten Trägerkatalysator enthält.

Die Hydrierung kann diskontinuierlich, bevorzugt aber kontinuierlich durchgeführt werden.

Die nach dem erfindungsgemäßen Verfahren in Schritt b) abgetrennte wässrige Phase enthält als Hauptprodukte 6-Hydroperoxycapronsäure, 6-Hydroxycapronsäure, 5-Hydroxyvaleriansäure, 1,2- und 1,4-Cyclohexandione, 1,2-und 1,4-Cyclohexandiole, Carbonsäuren mit einem bis sechs Kohlenstoffatomen, Adipinsäure, Bernsteinsäure, Glutarsäure und 5-Formylvaleriansäure. Diese wässrige Phase kann direkt für die katalytische Hydrierung zur Herstellung von 6-Hydroxycapronsäure verwendet werden.

Restmengen Cyclohexylhydroperoxid, Cyclohexanol und Cyclohexanon, die gegebenenfalls bei der Phasentrennung in Schritt b) des erfindungsgemäßen Verfahrens aus der wässrigen Phase nicht abgetrennt wurden, lassen sich jedoch gegebenenfalls durch nachträgliche Extraktion entfernen. Als Extraktionsmittel können aliphatische, cycloaliphatische oder aromatische Kohlenwasserstoffe wie n-Octan, Dodecan, Hexan, Toluol, Xylol, Cyclohexan, Methylcyclohexan, Cyclooctan, Cyclododecan oder Gemische dieser Verbindungen verwendet werden. Wird Cyclohexan als Extraktionsmittel eingesetzt, so kann der Extrakt direkt mit der Cyclohexanphase aus Schritt b) des erfindungsgemäßen Verfahrens vereinigt werden.

Eine weitere Möglichkeit, den Nebenprodukt-Anteil in der wässrigen Phase zu senken, besteht in der Konzentrierung der Wasserphase. Sie wird bei Temperaturen unter 50 °C und bei vermindertem Druck durchgeführt, um keine thermische Zersetzung der Hydroperoxycapronsäure herbeizuführen. Bei der Konzentrierung der Wasserphase fallen Dicarbonsäuren, insbesondere Adipinsäure aus der wässrigen Phase aus. Sie können durch Filtration oder Zentrifugierten abgetrennt werden. Einen Teil der Dicarbonsäuren und dabei vor allem Adipinsäure an dieser Stelle des Verfahrens abzutrennen, ist vorteilhaft. Andernfalls wird in Schritt d) des erfindungsgemäßen Verfahrens die gesamte Menge an Dicarbonsäuren mit niedermolekularen Alkoholen zu Dicarbonsäureestern umgesetzt.

Die anschließende destillative Abtrennung der Adipinsäurediester von entsprechenden 6-Hydroxycapronsäureestern in Schritt e) des erfindungsgemäßen Verfahrens ist jedoch mit hohem Energieaufwand verbunden.

Es ist jedoch auch möglich, 6-Hydroperoxycapronsäure und 6-Hydroxycapronsäure mit organischen Lösungsmitteln aus der wässrigen Phase zu extrahieren und dann die organischen Extrakte für die katalytische Hydrierung zu verwenden. Als unter den Hydrierbedingungen inerte Lösungsmittel kommen nach US-A 3 277 168 und DE-A 1 951 250, Seite 5 Alkanole mit vier bis zehn Kohlenstoffatomen, Cycloalkanole mit fünf bis acht Kohlenstoffatomen, Ester von Alkancarbonsäuren mit zwei bis acht Kohlenstoffatomen und Alkanolen mit einem bis acht Kohlenstoffatomen in Frage.

Es ist weiterhin möglich, die 6-Hydroperoxycapronsäure, wie im Fall von Cyclohexylhydroperoxid, durch Zugabe von Cobalt-Verbindungen zu 6-Hydroxycapronsäure zu deperoxidieren und das erhaltene Reaktionsgemisch anschließend zu hydrieren.

Es ist ebenfalls möglich, aus dem deperoxidierten Reaktionsgemisch 6-Hydroxycapronsäure und 5-Formylvaleriansäure zu extrahieren und den Extrakt einer Hydrierung zuzuführen.

Für die erfindungsgemäße Hydrierung sind 6-Hydroperoxycapronsäure und/oder 5-Formylvaleriansäure, gelöst in Wasser oder den als Extraktionsmitteln genannten organischen Lösungsmitteln geeignet.

Für die katalytische Hydrierung in Schritt c) des erfindungsgemäßen Verfahrens dienen Katalysatoren, die mindestens ein Metall ausgewählt aus Ruthenium und Rhenium enthalten.

Bevorzugt sind weiterhin Gemische oder Legierungen wie Palladium/Rhenium und Platin/Rhenium.

Gut geeignet sind weiterhin so genannte Vollkatalysatoren, die keine Träger enthalten und aus Metallen, Metalloxiden oder deren Gemischen bestehen.

Die Metalle oder Metallverbindungen können ohne Träger verwendet werden. Bevorzugt werden sie jedoch auf Träger, wie z. B. TiO₂ Al₂O₃, ZrO₂, SiO₂, HfO₂ Kohle, Zeolithe oder deren Gemische aufgebracht. Diese Trägerkatalysatoren können in verschiedensten Konfektionierungsformen, wie z. B. Strängen, Tabletten oder Ringen verwendet werden.

Besonders geeignet für die Hydrierung in Schritt c) des erfindungsgemäßen Verfahrens ist weiterhin ein Katalysator, enthaltend auf Titandioxid-Formkörpem geträgertes Ruthenium, wobei die Titandioxid-Formkörper durch Behandeln von Titandioxid vor oder nach dem Formen zum Formkörper mit 0,1 bis 30 Gew.-% einer Säure, in der Titandioxid schwer löslich ist, erhalten werden.

Das katalytisch aktive Ruthenium wird nach an sich bekannten Verfahren, bevorzugt auf vorgefertigtes TiO₂ als Trägermaterial aufgebracht.

Ein für die Verwendung in dem Ruthenium enthaltenden Katalysator bevorzugt geeigneter Titandioxid-Träger kann entsprechend DE-A 197 38 464 durch Behandeln von Titandioxid vor oder nach dem Formen des Formkörpers mit 0,1 bis 30 Gew.-% einer Säure, bezogen auf Titandioxid, in der das Titandioxid schwer löslich ist, erhalten werden. Bevorzugt wird Titandioxid in der Anatas-Modfikation verwendet. Als derartige Säure sind beispielsweise Ameisensäure, Phosphorsäure, Salpetersäure, Essigsäure oder Stearinsäure geeignet.

Die Aktivkomponente Ruthenium kann in Form einer Rutheniumsalzlösung auf den so erhaltenen Titandioxidträger in einer oder mehreren Tränkstufen aufgebracht werden.

Anschließend wird der getränkte Träger getrocknet und gegebenenfalls calciniert. Es ist jedoch auch möglich Ruthenium aus einer Rutheniumsalzlösung, bevorzugt mit Natriumcarbonat, auf einen als Pulver in wässriger Suspension vorliegende Titandioxids zu fällen. Die ausgefällten Niederschläge werden gewaschen, getrocknet, gegebenenfalls calciniert und verformt. Weiterhin können flüchtige Rutheniumverbindungen, wie beispielsweise Rutheniumacetylacetonat oder Rutheniumcarbonyl, in die Gasphase überführt werden und in an sich bekannter Weise auf den Träger aufgebracht werden, was als Chemical vapor deposition bezeichnet wird.

Die so erhaltenen, geträgerten Katalysatoren können in allen bekannten Konfektionierungsformen vorliegen. Beispiele sind Stränge, Tabletten oder Granulate. Vor ihrer Verwendung werden die Rutheniumkatalysatorvorläufer durch Behandlung mit Wasserstoffhaltigem Gas, bevorzugt bei Temperaturen über 100°C reduziert. Bevorzugt werden die Katalysatoren vor ihrem Einsatz im erfindungsgemäßen Verfahren bei Temperaturen von 0 bis 50°C, bevorzugt bei Raumtemperatur, mit sauerstofflialtigen Gasgemischen, bevorzugt mit Luft-Stickstoffgemischen, passiviert. Es ist auch möglich, den Katalysator in oxidischer Form in den Hydrierreaktor einzubauen und unter Reaktionsbedingungen zu reduzieren.

Der erfindungsgemäß besonders bevorzugte Katalysator weist einen Rutheniumgehalt von 0,1 bis 10 Gew.-%, bevorzugt von 2 bis 6, bezogen auf das Gesamtgewicht des Katalysators aus katalytisch aktivem Metall und Träger, auf. Der erfindungsgemäße Katalysator kann einen Schwefelgehalt von 0,01 bis 1 Gew.-%, bezogen auf das Gesamtgewicht des Katalysators, aufweisen, wobei die Schwefel-Bestimmung coulometrisch erfolgt.

Die Rutheniumoberfläche beträgt dabei von 1 bis 20 m2/g, bevorzugt von 5 bis 15 und die BET-Oberfläche (bestimmt nach DIN 66 131) von 5 bis 500 m2/g, bevorzugt von 50 bis 200 m2/g.

Die erfindungsgemäßen Katalysatoren weisen ein Porenvolumen von 0,1 bis 1 ml/g auf. Weiterhin zeichnen sich die Katalysatoren durch eine Schneidhärte von 1 bis 100 N aus.

Die Hydrierkatalysatoren können im Reaktionsgemisch suspendiert sein. Bevorzugt ist, sie im Hydrierreaktor fest anzuordnen. Die Hydrierung kann diskontinuierlich oder bevorzugt kontinuierlich durchgeführt werden. Das Reaktionsgemisch kann dabei in Sumpf- oder Rieselfahrweise über den Katalysator geleitet werden.

Im Ausgangsgemisch der Hydrierung sind zwei unterschiedliche Verbindungen, 6-Hydroperoxycapronsäure und 5-Formylvaleriansäure enthalten, die beide zu 6-Hydroxycapronsäure hydriert werden sollen. Da im einen Fall eine Hydroperoxy-, im andern Fall eine Aldehydgruppe hydriert werden muss, unterscheiden sich die optimalen Hydrierbedingungen beider Verbindungen.

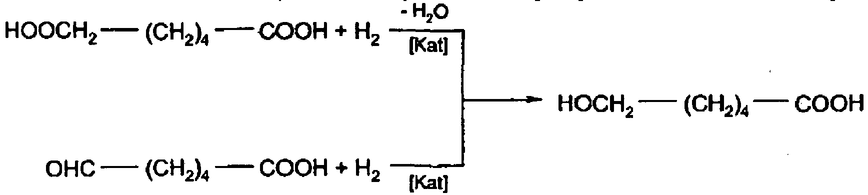

Da die Hydroperoxycapronsäure auch rein thermisch, aber weniger selektiv als bei einer Hydrierung, in 6-Hydroxycapronsäure übergehen kann, kann sie gemäß DE-A 1 951 250 in Gegenwart von Palladium-, Rhodium- oder Platin-Katalysatoren bei 15 bis 130 °C, vorzugsweise 50 bis 100 °C, also bei moderaten Temperaturen hydriert.

Das Vergleichsbeispiel zeigt, dass Aldehydgruppen unter den Bedingungen der 6-Hydroperoxycapronsäure-Hydrierung in DE-A 1 951 250 nicht oder nur wenig hydriert werden. Hierfür sind höhere Temperaturen und Drucke notwendig.

Die Hydrierung in Schritt c) des erfindungsgemäßen Verfahrens kann in einem einzigen Reaktor oder in zwei hintereinander geschalteten Reaktoren durchgeführt werden. Verwendet man zwei Reaktoren, so können die beiden Reaktoren den gleichen Katalysator oder zwei verschiedene Katalysatoren enthalten. Dabei können sich die beiden Reaktoren in der Hydriertemperatur und dem Wasserstoffpartialdruck unterscheiden.

Es ist weiterhin möglich, die Hydrierung in einem einzigen Reaktor, der miteinem einzigen Katalysator gefüllt ist, so durchzuführen, dass die Hydriertemperatur im Reaktor innerhalb eines gewünschten Temperaturbereichs ansteigt.

Die Hydrierung in Schritt c) des erfindungsgemäßen Verfahrens erfolgt unabhängig davon ob in Schritt a) in An- oder Abwesenheit eines Katalysators gearbeitet wurde bei Temperaturen von über 100 bis 200 °C, bevorzugt 120 bis 180 °C, besonders bevorzugt 130 bis 170 °C. Der Wasserstoff-Partialdruck beträgt dabei 1 bis 100 bar, bevorzugt 2 bis 80 bar, besonders bevorzugt 5 bis 60 bar.

In Schritt a) des erfindungsgemäßen Verfahrens wird ein Ausgangsgemisch verwendet, das ohne Katalysator oxidiert wurde. Der Einsatz von Katalysatoren ist jedoch auch in Schritt a) möglich. Werden Katalysatoren wie z.B. Cobaltcarboxylate eingesetzt so entstehen neben 6-Hydroxycapronsäure nur geringe Mengen an 6-Hydroperoxycapronsäure. In diesem Falle erfolgt dann die Hydrierung von überwiegend der 5-Formylvaleriansäure nach Schritt c) des erfindungsgemäßen Verfahrens.

Für die zur Veresterung nach Schritt d) des erfindungsgemäßen Verfahrens umzusetzenden Carbonsäuren - 6-Hydroxycapronsäure und α, ω- Dicarbonsäuren mit vier bis sechs Kohlenstoffatomen - kommen in der Regel Alkanole mit 1 bis 12 C-Atomen, Cycloalkanole mit 5 bis 7 C-Atomen, Aralkanole mit 7 bis 8 C-Atomen oder Phenole mit 6 bis 9 C-Atomen in Betracht. Dabei können Methanol, Ethanol, Propanol, iso-Propanol, n- oder i-Butanol oder auch n-Pentanol oder i-Pentanol oder Gemische der Alkohole, bevorzugt aber Alkohole mit 1 bis 4 C-Atomen, besonders bevorzugt Methanol verwendet werden. Die Estergruppen in den 6-Hydroxycapronsäureestern und den Adipinsäurediestern können gleich oder verschieden sein, bevorzugt sind sie jedoch gleich.

Das aus Schritt c) des erfindungsgemäßen Verfahrens erhaltene Produkt ist im Allgemeinen eine wässrige Lösung mit einem Wasseranteil von 20 bis 80 %. Da eine Veresterungsreaktion eine Gleichgewichtsreaktion darstellt, bei der Wasser entsteht, ist es sinnvoll, insbesondere bei Veresterung mit z.B. Methanol, vorhandenes Wasser vor der Reaktion zu entfernen, vor allem wenn während der Veresterungsreaktion Wasser nicht, z.B. nicht azeotrop, entfernt werden kann. Die Entwässerung kann z.B. mit einem Membransystem erfolgen, oder bevorzugt in einer Destillationsapparatur, bei der bei 10 bis 250°C, bevorzugt 20 bis 200°C, besonders bevorzugt 30 bis 200°C und einem Druck von 1 bis 1500 mbar, bevorzugt 5 bis 1100 mbar, besonders bevorzugt 20 bis 1000 mbar Wasser über Kopf und höhere Dicarbonsäuren über Sumpf abgetrennt werden. Die Sumpftemperatur wird dabei bevorzugt so gewählt, dass das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.-% betragen.

Dem entwässerten Hydrieraustrag wird ein Alkohol mit 1 bis 10 C-Atomen zugemischt. Dabei können Methanol, Ethanol, Propanol oder iso-Propanol oder Gemische der Alkohole, bevorzugt aber Methanol einerseits oder C₄- und höhere Alkohole, insbesondere mit 4 bis 8 C-Atomen und bevorzugt n- oder i-Butanol oder auch n-Pentanol oder i-Pentanol andererseits verwendet werden. Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor, in dem die Carbonsäuren mit dem Alkohol verestert werden. Die Veresterungsreaktion kann bei 50 bis 400°C, bevorzugt 70 bis 300°C, besonders bevorzugt 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen, bevorzugt wird aber zur Erhöhung der Reaktionsgeschwindigkeit ein Katalysator zugesetzt. Dabei kann es sich um einen homogenen gelösten oder um einen festen Katalysator handeln. Als homogene Katalysatoren seien beispielhaft Schwefelsäure, Phosphorsäure, Salzsäure, Sulfonsäuren wie p-Toluolsulfonsäure, Heteropolysäuren wie Wolframatophosphorsäure oder Lewissäuren wie z. B. Aluminium-, Vanadium-, Titan-, Bor-Verbindungen genannt. Bevorzugt sind Mineralsäuren, insbesondere Schwefelsäure. Das Gewichtsverhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie SiO₂, Al₂O₃, SnO₂, ZrO₂, Schichtsilikate oder Zeolithe, die alle zur Säureverstärkung mit Mineralsäureresten wie Sulfat oder Phosphat dotiert sein können, oder organische lonentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich, z.B. durch eine Membran oder destillativ entfernt.

Wurde die Hydrierung in Schritt c) des erfindungsgemäßen Verfahrens in einem organischen Lösungsmittel (Extraktionsmittel für 6-Hydroxycapronsäure), z. B. Methylcyclohexan, durchgeführt, so kann die Veresterung in diesem Lösungsmittel durchgeführt und das Reaktionswasser nach Phasentrennung abgetrennt werden.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt abzüglich der gegebenenfalls zugesetzten Säure als Katalysator 0,01 bis 50, bevorzugt 0,1 bis 10 mg/KOH. Dabei müssen nicht alle im System vorhandenen Carboxylgruppen als Ester des eingesetzten Alkohols vorliegen, sondern ein Teil kann in Form von dimeren oder oligomeren Estern mit dem OH-Ende der Hydroxycapronsäure vorliegen.

Das Veresterungsgemisch aus Schritt d) des erfindungsgemäßen Verfahrens wird in ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet. Es kann jedoch auch mit basischen lonentausehern neutralisiert werden.

Wird eine Kolonne verwendet, so erfolgt der Zulauf zur Kolonne bevorzugt zwischen dem Kopf- und dem Sumpfstrom. Über Kopf wird bei Drücken von 1 bis 1500 mbar, bevorzugt 20 bis 1000 mbar, besonders bevorzugt 40 bis 800 mbar und Temperaturen zwischen 0 und 150°C, bevorzugt 15 und 90°C und insbesondere 25 und 75°C der überschüssige Veresterungsalkohol, Wasser und Leichtsieder abgezogen. Leichtsieder sind dabei Komponenten, die einen geringeren Siedpunkt aufweisen wie 6-Hydroxycapronsäure- und Adipinsäurediester.

Als Sumpf wird ein Estergemisch erhalten, das vorwiegend den Estern des eingesetzten Alkohols mit Dicarbonsäuren wie Adipinsäure und Glutarsäure, Hydroxycarbonsäuren wie 6-Hydroxycapronsäure, sowie Oligomere und freien bzw. veresterten 1,2- und 1,4-Cyclohexandiolen enthält. Es kann sinnvoll sein, einen Restgehalt von Wasser und/oder Alkohol bis je 4 Gew.-% im Estergemisch zuzulassen. Die Sumpftemperaturen betragen 70 bis 250oC. bevorzugt 80 bis 220°C, besonders bevorzugt 100 bis 190°C.

Der weitgehend von Wasser und Veresterungsalkohol befreite Strom wird in eine weitere Destillationskolonne eingespeist. Die Kolonne wird bei Temperaturen von 10 bis 300°C, bevorzugt 20 bis 270°C, besonders bevorzugt 30 bis 250°C und Drucken von 1 bis 1 000 mbar, bevorzugt 5 bis 500 mbar, besonders bevorzugt 10 bis 200 mbar betrieben.

Die Kopffraktion besteht überwiegend aus Restwasser und Restalkohol, Estern des Alkohols mit Monocarbonsäuren, überwiegend C₃- bis C₆-Monocarbonsäureestern mit Hydroxycarbonsäuren, wie 6-Hydroxycapronsäure, 5-Hydroxyvaleriansäure sowie vor allem den Diestem mit Dicarbonsäuren wie Adipinsäure, Glutarsäure und Bernsteinsäure, weiterhin Cyclohexandiolen, Caprolacton und Valerolaceton.

Die genannten Komponenten können zusammen über Kopf abgetrennt oder in einer weiteren bevorzugten Ausführungsform in einer Kolonne der Stufe 4 in einen Kopfstrom, der überwiegend Restwasser und Restalkohol sowie die oben erwähnten Bestandteile mit 3 bis 5 C-Atomen enthält und einen Seitenstrom, der überwiegend die oben erwähnten Bestandteile der C₆-Ester enthält, aufgetrennt werden.

Die schwersiedenden Komponenten mit einer Siedetemperatur über denen von 6-Hydroxycapronsäure- und Adipinsäurediestern, überwiegend enthaltend dimere oder oligomere Ester, Cyclohexandiole sowie nicht näher definierten z. T. polymeren Bestandteilen, werden über den Abtriebsteil der Kolonne abgetrennt.

Für die Caprolactonherstellung wird der vorwiegend Ester der C₆-Säuren enthaltende Strom aus dem Schritt d) des erfindungsgemäßen Verfahrens eingesetzt. Dazu wird dieser Strom einer Destillationskolonne, in einen überwiegend Adipinsäurediester enthaltenden Strom über Kopf und einen überwiegend 6-Hydroxycapronsäureester enthaltenden Strom über Sumpf aufgetrennt. Die Kolonne wird bei Drücken von 1 bis 500 mbar, bevorzugt 5 bis 350 mbar, besonders bevorzugt 10 bis 200 mbar und Sumpftemperaturen von 80 bis 250°C, bevorzugt 100 bis 200°C, besonders bevorzugt 110 bis 180°C betrieben. Die Kopftemperaturen stellen sich dabei entsprechend ein.

Wichtig für eine hohe Reinheit und hohe Ausbeute an Caprolacton ist die Abtrennung der 1,2-Cyclohexandiole vom 6-Hydroxycapronsäureester, da diese Komponenten Azeotrope miteinander bilden.

Der 0 bis 40 Gew.-% Adipinsäurediester aufweisende 6-Hydroxycapronsäureester-Strom kann in der Gasphase oder der Flüssigphase zu Alkohol und Caprolacton umgesetzt werden.

Das erfindungsgemäße Verfahren zur Herstellung von 6-Hydroxycapronsäureestern besitzt eine Reihe von Vorteilen gegenüber dem Stand der Technik:
Die Hydrierung von 5-Formylvaleriansäure steigert die 6-Hydroxycapronsäure-Ausbeute, da sonst nur aus der 6-Hydroxycapronsäure der entsprechende Ester erhalten wurde. Ein weiterer Vorteil liegt darin, dass bei dem erfindungsgemäßen Verfahren nicht mehr die 6-Hydroxycapronester von 5-Formylvalerlanestern abgetrennt werden muss.

Außerdem lässt sich das erfindungsgemäße Verfahren gegenüber dem Stand der Technik dadurch vereinfachen, dass sich ein Teil der Adipinsäure nach Abdampfen von Wasser aus der wässrigen Lösung auskristallisieren lässt. Eine weitere vorteilhafte Möglichkeit besteht darin, 6-Hydroperoxy- und 6-Hydroxycapronsäure durch Extraktion mit organischen Lösungsmitteln von Adipinsäure abzutrennen. Damit wird die destillative 6-Hydroxycapronester/Adipinsäurediester-Trennung entlastet.

### Beispiele (Die Analysen wurden mittels Gaschromatographie durchgeführt):

Beispiel 1 (nicht erfindungsgemäß): Analog dem in DE-A 1 951 250 Beispiel 1 beschriebenen Verfahren wurden 100 g des nach 1b der Patentschrift erhaltene Produktes, das It. gaschromatographischer Analyse noch 4,5 % 5-Formylvaleinansäure enthielt, in Wasser gelöst (50 Gew.-% Anteil) und bei 130 °C und 35 bar Wasserstoffdruck in einem gerührten Autoklaven an einem zuvor mit Wasserstoff bei 250 °C aktivierten Katalysator hydriert (Katalysatorzusammensetzung in oxidischer Form: 23 % NiO, 8 % CuO, 2 % Mn₂O₃ auf SiO₂). Nach 10 h Reaktionszeit war der Gehalt (wasserfrei gerechnet) an 5-Formylvaleriansäure auf 0,1 % abgesunken, der Gehalt an 6-Hydroxycapronsäure entsprechend um ca. 4 % auf ca. 64 % gestiegen. Alle anderen Komponenten blieben nahezu unverändert. Dieses Hydrieraustragsgemisch wurde per Destillation von Wasser befreit, mit 0,2 % Schwefelsäure und 200 g Methanol versetzt und 5 h unter Eigendruck auf 130 °C erhitzt. Nach Abkühlen wurde die Schwefelsäure mit äquimolaren Mengen Natriumcarbonat neutralisiert und der Ansatz fraktioniert aufdestilliert. Bei Normaldruck wurde zuerst Methanol, dann Wasser entfernt, danach erhielt man bei 10 mbar mehrere Fraktionen, die 6-Hydroxycapronsäuremethylester enthielten, mit einer Reinheit von bis zu 99,2 %.

Beispiel 1 wird wiederholt, mit dem Unterschied, dass das wässrige Extrakt nach DE-A 1 951 250 Beispiel 1 a) (Hergestellt wie in französischer Patentschrift 1 491 518 beschrieben) in die Hydrierung eingesetzt wurde. Im Hydrieraustrag fanden sich 0,2 % 5-Formylvaleriansäure aber keine 6-Hydroperoxycapronsäure mehr. Der Gehalt an 6-Hydroxycapronsäure lag wie in Beispiel 1 bei ca. 64 %. Weitere Ergebnisse siehe Tab. 1.

Beispiel 2 wurde wiederholt. Als Hydrierkatalysator wurde ein Katalysator eingesetzt, der im oxidischen Zustand folgende Zusammensetzung aufweist: 66 % CoO, 20 % CuO, 7 % Mn₂O₃, weitere Komponenten Alkali- und Erdalkali- sowie Phosphoroxide. Die Hydrierung erfolgte bei 150 °C. Ergebnisse siehe Tab. 1

Beispiel 2 wurde wiederholt. Als Hydrierkatalysator wurde ein Ru (5 % als Oxid gerechnet) auf Aktivkohle eingesetzt. Die Hydrierung erfolgte bei 130 °C. Ergebnisse siehe Tab. 1

Beispiel 2 wurde wiederholt. Als Hydrierkatalysator wurde ein Re (6 % als Oxid gerechnet) auf Aktivkohle eingesetzt. Die Hydrierung wurde bei 155 °C durchgeführt. Ergebnisse siehe Tab. 1

**Tabelle 1:**

| Beispiel | Restgehalt 5-Formylvaleriansäure nach Hydrierung (%) | Reinheit 6-Hydroxycapronsäuremethylester (%) |
|---|---|---|
| 2^{*)} | 0,1 | 99,1 |
| 3^{*)} | 0,02 | 99,4 |
| 4 | < 0,02 | 99,5 |
| 5 | 0,08 | 99,0 |
| *) = nicht erfindungsgemäß | | |

### Vergleichsbeispiel 1:

Beispiel 2 wurde wiederholt, wobei an einem Pd (5 % als PdO) auf Kohle-Katalysator hydriert wurde. Der Gehalt an Hydroperoxid war danach Null, jedoch war der Gehalt an 5-Formylvaleriansäure nur auf 3,9 % gesunken. Nach Veresterung und Destillation wurde ein 6-Hydroxycapronsäuremethylester mit einer maximalen Reinheit von 98,1 % erhalten.

### Vergleichsbeispiel 2:

Beispiel 1 aus DT-OS 1 951 250 wurde wiederholt. Der Ausgangsgehalt vor der Hydrierung an 5-Formylvaleriansäure von 4,5 % war nach der Hydrierung unverändert.

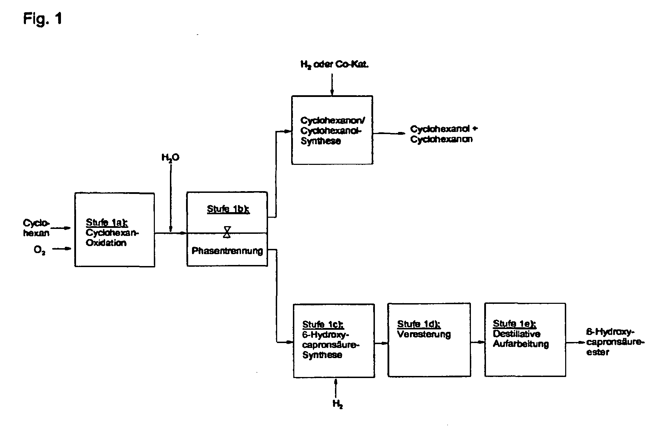

## Patentansprüche

1. Verfahren zur Herstellung von 6-Hydroxycapronsäureestern, wobei man
a) Cyclohexan mit molekularem Sauerstoff oder Gemischen aus molekularem Sauerstoff und unter den Reaktionsbedingungen inerten Gasen zu einem Reaktionsgemisch oxidiert, das als Hauptkomponenten Cyclohexylhydroperoxid, Cyclohexanol, Cyclohexanon, unumgesetztes Cyclohexan, 6-Hydroperoxycapronsäure, 6-Hydroxycapronsäure, 5-Formylvaleriansäure und α, ω- Dicarbonsäuren mit vier bis sechs Kohlenstoffatomen enthält,
b) das Reaktionsgemisch aus Schritt a) nach Zugabe von Wasser in eine organische, Cyclohexan und die Cyclohexan-Verbindungen enthaltende und eine wässrige, die Carbonsäuren enthaltende Phase trennt,
c) die wässrige Phase aus b) katalytisch hydriert,
d) die in der wässrigen Phase enthaltenen Carbonsäuren mit einem 1 bis 10 C-Atome enthaltendem Alkohol zu den entsprechenden Carbonsäureestern umsetzt und
e) aus dem Veresterungsgemisch aus Schritt d) durch Destillation 6-Hydroxycapronsäureester gewinnt,
wobei bei der katalytischen Hydrierung in Schritt c) mindestens 5-Formylvaleriansäure zu 6-Hydroxycapronsäure hydriert wird und als Katalysatormetall für die Hydrierung in Schritt c) mindestens ein Metall, ausgewählt aus der Gruppe von Ruthenium und Rhenium verwendet wird.

2. Verfahren nach dem Anspruch 1, wobei der Schritt a) in Anwesenheit eines Katalysators durchgeführt wird.

3. Verfahren nach dem Anspruch 1, wobei der Schritt a) in Abwesenheit eines Katalysators durchgeführt wird.

4. Verfahren nach einem der Ansprüche 1 bis 3, wobei man in Schritt c) neben 5-Formylvaleriansäure auch 6-Hydroperoxycapronsäure zu 6-Hydroxycapronsäure hydriert.

5. Verfahren nach einem der Ansprüche 1 bis 4, wobei man die wässrige, die Carbonsäuren enthaltende Phase aus Schritt b) zur Entfernung von Restmengen Cyclohexylhydroperoxid, Cyclohexanol und Cyclohexanon mit einem aliphatischen, cycloaliphatischen oder aromatischen Kohlenwasserstoff extrahiert.

6. Verfahren nach einem der Ansprüche 1 bis 5, wobei man 6-Hydroperoxycapronsäure und 6-Hydroxycapronsäure aus der wässrigen Phase von Schritt b) mit einem organischen, inerten Lösungsmittel aus der Wasserphase extrahiert.

7. Verfahren nach einem der Ansprüche 1 bis 6, wobei die wässrige, die Carbonsäuren enthaltende Phase aus Schritt b) durch Abdestillieren von Wasser konzentriert wird und die in fester Form ausfallenden Carbonsäuren abgetrennt werden.

8. Verfahren nach einem der Ansprüche 1 bis 7, wobei man die Hydrierung in Schritt c) bei Temperaturen von 100 bis 200°C und Drücken von 1 bis 100 bar durchführt.

## Claims

1. A process for preparing 6-hydroxycaproic esters, wherein
a) cyclohexane is oxidized with molecular oxygen or mixtures of molecular oxygen and gases which are inert under the reaction conditions to give a reaction mixture which comprises, as main components, cyclohexyl hydroperoxide, cyclohexanol, cyclohexanone, unconverted cyclohexane, 6-hydroperoxycaproic acid, 6-hydroxycaproic acid, 5-formylvaleric acid and α,ω-dicarboxylic acids having from four to six carbon atoms,
b) the reaction mixture from step a), after adding water, is separated into an organic phase comprising cyclohexane and the cyclohexane compounds, and an aqueous phase comprising the carboxylic acids,
c) the aqueous phase from b) is catalytically hydrogenated,
d)the carboxylic acids present in the aqueous phase are reacted with an alcohol comprising from 1 to 10 carbon atoms to give the corresponding carboxylic esters and
e) 6-hydroxycaproic esters are obtained by distillation from the esterification mixture from step d),
the catalytic hydrogenation in step c) involving at least hydrogenation of 5-formylvaleric acid to 6-hydroxycaproic acid and the catalyst metal used for the hydrogenation in step c) being at least one metal selected from the group of ruthenium and rhenium.

2. The process according to claim 1, wherein step a) is performed in the presence of a catalyst.

3. The process according to claim 1, wherein step a) is performed in the absence of a catalyst.

4. The process according to any one of claims 1 to 3, wherein not only 5-formylvaleric acid but also 6-hydroperoxycaproic acid is hydrogenated in step c) to 6-hydroxycaproic acid.

5. The process according to any one of claims 1 to 4, wherein the aqueous phase comprising the carboxylic acids from step b), to remove residual amounts of cyclohexyl hydroperoxide, cyclohexanol and cyclohexanone, is extracted with an aliphatic, cycloaliphatic or aromatic hydrocarbon.

6. The process according to any one of claims 1 to 5,
wherein 6-hydroperoxycaproic acid and 6-hydroxycaproic acid are extracted from the aqueous phase of step b) with an organic, inert solvent from the water phase.

7. The process according to any one of claims 1 to 6, wherein the aqueous phase comprising the carboxylic acids from step b) is concentrated by distilling water off and the carboxylic acids which precipitate out in solid form are removed.

8. The process according to any one of claims 1 to 7, wherein the hydrogenation in step c) is performed at temperatures of from 100 to 200°C and pressures of from 1 to 100 bar.

## Revendications

1. Procédé pour la production d'esters d'acide 6-hydroxycaproïque, dans lequel
a) on soumet à une oxydation du cyclohexane avec de l'oxygène moléculaire ou des mélanges d'oxygène moléculaire et de gaz inertes dans les conditions réactionnelles, pour obtenir un mélange réactionnel qui contient comme composants principaux de l'hydroperoxyde de cyclohexyle, du cyclohexanol, de la cyclohexanone, du cyclohexane n'ayant pas réagi, de l'acide 6-hydroperoxy-caproïque, de l'acide 6-hydroxycaproïque, de l'acide 5-formylvalérique et des acides α,ω-dicarboxyliques ayant de quatre à six atomes de carbone,
b) on partage le mélange réactionnel provenant de l'étape a), après addition d'eau, en une phase organique, contenant du cyclohexane et les composés de cyclohexane et une phase aqueuse contenant les acides carboxyliques,
c) on soumet la phase aqueuse provenant de b) à une hydrogénation catalytique,
d) on fait réagir les acides carboxyliques contenus dans la phase aqueuse avec un alcool contenant de 1 à 10 atomes de carbone, pour obtenir les esters d'acides carboxyliques correspondants et
e) on obtient des esters d'acide 6-hydroxycaproïque par distillation à partir du mélange d'estérification provenant de l'étape d),
lors de l'hydrogénation catalytique dans l'étape c) au moins de l'acide 5-formylvalérique étant hydrogéné en acide 6-hydroxycaproïque et pour l'hydrogénation dans l'étape c) on utilise au moins un métal, choisi dans le groupe constitué par le ruthénium et le rhénium.

2. Procédé selon la revendication 1, dans lequel l'étape a) est effectuée en présence d'un catalyseur.

3. Procédé selon la revendication 1, dans lequel l'étape a) est effectuée en absence d'un catalyseur.

4. Procédé selon l'une quelconque des revendications 1 à 3, dans lequel dans l'étape c) on soumet à une hydrogénation en acide 6-hydroxycaproïque outre de l'acide 5-formylvalérique également de l'acide 6-hydroperoxycaproïque.

5. Procédé selon l'une quelconque des revendications 1 à 4, dans lequel on extrait avec un hydrocarbure aliphatique, cycloaliphatique ou aromatique la phase aqueuse provenant de l'étape b), contenant les acides carboxyliques, afin d'éliminer des quantités résiduelles d'hydroperoxyde de cyclohexyle, de cyclohexanol et de cyclohexanone.

6. Procédé selon l'une quelconque des revendications 1 à 5, dans lequel on extrait de la phase aqueuse, à l'aide d'un solvant organique inerte, l'acide 6-hydroperoxycaproïque et l'acide 6-hydroxy-caproïque de la phase aqueuse de l'étape b).

7. Procédé selon l'une quelconque des revendications 1 à 6, dans lequel on concentre par élimination de l'eau par distillation la phase aqueuse provenant de l'étape b), contenant les acides carboxyliques, et on sépare les acides carboxyliques apparaissant sous forme solide.

8. Procédé selon l'une quelconque des revendications 1 à 7, dans lequel on effectue l'hydrogénation dans l'étape c) à des températures de 100 à 200 °C et sous des pressions de 1 à 100 bars.
